# EUROPÄISCHE PATENTSCHRIFT

(11) **EP 2 770 938 B1**
(45) Veröffentlichungstag und Bekanntmachung des Hinweises auf die Patenterteilung: **30.12.2015**
(21) Anmeldenummer: 12778322.3
(22) Anmeldetag: 23.10.2012
(51) Int. Cl.: A61B 19/02, A61L 2/28, A61L 2/26

(54) **MEDIZINISCHER STERILBEHÄLTER UND VERFAHREN ZUM BESTIMMEN DES STERILISATIONSSTATUS EINES MEDIZINISCHEN STERILBEHÄLTERS**
STERILE MEDICAL CONTAINER AND METHOD FOR DETERMINING THE STERILIZATION STATUS OF A STERILE MEDICAL CONTAINER
CONTENEUR DE STÉRILISATION MÉDICAL ET PROCÉDÉ PERMETTANT DE DÉTERMINER L'ÉTAT DE STÉRILISATION D'UN CONTENEUR DE STÉRILISATION MÉDICAL

(30) Priorität: 26.10.2011 DE 102011054827
(43) Veröffentlichungstag der Anmeldung: 03.09.2014
(73) Patentinhaber: Aesculap AG, 78532 Tuttlingen (DE)
(72) Erfinder: SCHUSTER, Stefan, 78048 Villingen-Schwenningen (DE)
(74) Vertreter: Hoeger, Stellrecht & Partner Patentanwälte mbB
(86) Internationale Anmeldenummer: PCT/EP2012/070944
(87) Internationale Veröffentlichungsnummer: WO 2013/060667

(56) Entgegenhaltungen:
- WO-A1-00/51648
- DE-A1- 3 632 674
- DE-C1- 19 835 503
- DE-U1-202009 010 210
- US-A- 5 508 006

## Beschreibung

Die vorliegende Erfindung betrifft einen medizinischen Sterilbehälter mit einem Behälterinnenraum zum Aufnehmen zu sterilisierender Gegenstände, welcher Sterilbehälter eine druckaktivierbare Sterilisationsstatusdetektionseinrichtung zum Detektieren eines Sterilisationsstatus des Sterilbehälters umfasst.

Ferner betrifft die vorliegende Erfindung ein Verfahren zum Bestimmen des Sterilisationsstatus eines medizinischen Sterilbehälters, welcher einen Behälterinnenraum zum Aufnehmen zu sterilisierender Gegenstände und eine druckaktivierbare Sterilisationsstatusdetektionseinrichtung umfasst, wobei, während der Sterilbehälter einen Sterilisationsprozess in einem Sterilisationsgerät durchläuft, Druckdifferenzen, denen der Sterilbehäiter dabei unterworfen ist, detektiert werden und dem Sterilbehälter der Sterilisationsstatus "steril" zugeordnet wird, wenn während des Sterilisationsprozesses ein Grenzwert für einen Druckgradient pro Zeiteinheit überschritten wird.

Medizinische Sterilbehälter, auch als Sterilisierbehälter oder Sterilcontainer bezeichnet, dienen insbesondere Medizinern und deren Hilfspersonal in der Chirurgie dazu, chirurgische Instrumente und Implantate auf einfache Weise zu sterilisieren und lagern. Zu diesem Zweck werden die zu sterilisierenden Gegenstände zunächst nach gründlicher Reinigung in den Behälterinnenraum eingebracht. Der Sterilbehälter wird nach dem Verschließen in einem Sterilisationsgerät, das auch als Sterilisator oder Autoklav bezeichnet wird, mit seinem Inhalt sterilisiert. Damit eine Sterilisation des Inhalts des Sterilbehälters im geschlossenen Zustand überhaupt möglich ist, weist dieser üblicherweise Gasaustauschöffnungen auf, die das Eindringen von Sattdampf zum Sterilisieren des Behälterinhalts in den Behälterinnenraum ermöglichen. Die Gasaustauschöffnungen können dauerhaft für einen Gasaustausch offen sein. In diesem Fall sind sie dann mit einer Sterilbarriere, beispielsweise einem Filter, vollständig verschlossen, wobei die Steril barriere jedoch einen Gasaustausch ermöglicht. Alternativ können Gasaustauschöffnungen auch mit Ventilen verschlossen sein, die sich nur dann, wenn sich der Sterilbehälter im Sterilisationsgerät befindet und einem Sterilisationsprozess unterzogen wird, öffnen, um Sattdampf in den Behälter strömen und Feuchtigkeit gegebenenfalls wieder aus diesem austreten lassen, und nach abgeschlossenem Prozess wieder automatisch die Gasaustauschöffnungen schließen.

Bei einem Sterilisationsprozess wird der Sterilbehälter in einem Sterilisationsgerät einer Heißdampfbehandlung unterzogen, bei welcher Sattdampf mit einer Temperatur von typischerweise 134° und einem Druck von circa 3,1 bar für eine vorgegebene Zeitdauer zwischen 3 und 60 Minuten beaufschlagt wird.

Nach komplettem Durchlaufen des Sterilisationsprozesses im Sterilisationsgerät kann dem Sterilbehälter, das heißt seinem Innenraum und den darin enthaltenen Gegenständen, der Sterilisationsstatus "steril" zugeordnet werden.

Bei am Markt erhältlichen Sterilbehältern ist es bekannt, den Sterilisationsstatus automatisch auf "steril" zu setzen und entsprechend anzuzeigen, wenn während des Sterilisationsprozesses eine Grenztemperatur überschritten wird. Bekannt sind beispielsweise optische Anzeigen, die nach Überschreiten einer Temperatur von beispielsweise 115° ihre Farbe ändern und damit einem Verwender anzeigen, dass der Sterilbehälter vermeintlich einen Sterilisationszyklus durchlaufen hat.

Nachteilig bei derartigen Systemen ist jedoch, dass sie nur auf Temperaturänderungen reagieren. Mit anderen Worten würde einem Sterilbehälter auch der Sterilisationsstatus "steril" zugeordnet, wenn zwar die vorgegebene Temperatur überschritten, jedoch kein Sattdampf zur Sterilisation der im Behälter enthaltenen Gegenstände in den Behälter eingeströmt wäre.

Da die Validierung des Sterilisationsprozesses, insbesondere in Krankenhäusern, von großer Bedeutung ist, sind sämtliche Arten von Anzeigen des Sterilisationsstatus inakzeptabel, die einem Sterilbehälter den Sterilisationsstatus "steril" zuordnen, wenn nicht eindeutig sichergestellt ist, dass dieser Sterilisationsstatus auch tatsächlich vorliegt. Eine temperaturaktivierte Sterilisationsstatusdetektiön ist folglich nicht geeignet, um mit absoluter Sicherheit festzustellen, dass der Sterilbehälter und dessen Inhalt einen Sterilisationsprozess ordnungsgemäß durchlaufen haben.

Sterilbehälter und Verfahren zum Bestimmen des Sterilisationsstatus eines medizinischen Sterilbehälters sind insbesondere aus der DE 36 32 674 A1 bekannt. Weitere Sterilbehälter sind in der DE 20 2009 010 210 U1, der US 5,508,006, der WO 00/51648 A1 und der DE 198 35 503 C1 beschrieben.

Es ist daher Aufgabe der vorliegenden Erfindung, einen medizinischen Sterilbehälter sowie ein Verfahren der eingangs beschriebenen Art so zu verbessern, dass die Zuverlässigkeit bei der Zuordnung des Sterilisationsstatus "steril" verbessert wird.

Diese Aufgabe wird bei einem medizinischen Sterilbehälter der eingangs beschriebenen Art erfindungsgemäß dadurch gelöst, dass die Sterilisationsstatusdetektionseinrichtung eine Kolbenzylinderanordnung umfasst.

Eine druckaktivierbare Sterilisationsstatusdetektionseinrichtung vorzusehen hat gegenüber einer temperaturaktivierbaren Sterilisationsstatusdetektionseinrichtung den Vorteil, dass mit praktisch absoluter Sicherheit einem Sterilbehälter der richtige Sterilisationsstatus zugeordnet werden kann. Die druckaktivierbare Sterilisationsstatusdetektionseinrichtung ermöglicht es insbesondere, Druckänderungen beim Durchlaufen eines Sterilisationsprozesses zu nutzen und von diesen auf das ordnungsgemäße Durchlaufen eines solchen Sterilisationsprozesses zu schließen. Insbesondere nach der eigentlichen Sterilisation mit Heißdampf unter hohem Druck und Temperaturen über 100°, wird zum Austrocknen der Autoklav evakuiert, was einen signifikanten Druckabfall zur Folge hat. Bei Kenntnis des Druckverlaufs in der Kammer des Autoklaven kann somit die Sterilisationsdetektionseinrichtung druckaktiviert werden und dem Sterilbehälter nach korrektem Durchlauf eines Sterilisationsprozesses automatisch den Sterilisationsstatus "steril" zuordnen. Ferner lässt sich eine druckaktivierbare Sterilisationsstatusdetektionseinrichtung auch bei anderen Sterilisationsverfahren einsetzen, beispielsweise bei der Gasplasmasterilisation. Temperaturaktivierte Systeme funktionieren jedoch nur in der Dampfsterilisation. Die Kolbenzylinderanordnung ermöglicht es, Druckänderungen in eine Bewegung des Kolbens umzusetzen, um so beispielsweise eine Anzeige zu betätigen oder eine Verschlusseinrichtung zu verriegeln. Selbstverständlich ist es alternativ auch denkbar, dass die Sterilisationsstatusdetektionseinrichtung jede andere Art von elektrischer oder elektromechanischer Druckmesseinrichtung umfasst. Eine vollmechanische Kolbenzylinderanordnung hat jedoch gegenüber elektrischen Drucksensoren den Vorteil, dass keine elektrische Energie zu deren Betrieb benötigt wird, so dass beim Vorsehen einer Kolbenzylinderanordnung vollständig auf Batterien oder eine netzabhängige Energieversorgung verzichtet werden kann.

Um die Handhabbarkeit des Sterilbehälters zu verbessern, ist es vorteilhaft, wenn er ein Behälterunterteil und ein Behälteroberteil zum Verschließen des Behälterunterteils in einer Schließstellung des Sterilbehälters umfasst. Das Behälterunterteil kann insbesondere wannenförmig ausgebildet sein, das Behälteroberteil in Form eines Deckels. Optionale Gasaustauschöffnungen des Sterilbehälters können wahlweise am Behälterunterteil und/oder am Behälteroberteil vorgesehen sein, und mit Ventilen und/oder Keimbarrieren, beispielsweise Filtern, temporär oder dauerhaft geöffnet werden.

Je nach Form, Größe und Einsatzzweck des Sterilbehälters kann es günstig sein, wenn die Sterilisationsstatusdetektionseinrichtung am Behälterunterteil und/oder am Behälteroberteil angeordnet oder ausgebildet ist. Denkbar ist es insbesondere, die Sterilisationsstatusdetektionseinrichtung zwei- oder mehrteilig auszubilden, wobei ein Teil derselben am Behälterunterteil und ein anderer Teil derselben am Behälteroberteil angeordnet oder ausgebildet werden kann, so dass sie erst dann als Sterilisationsstatusdetektionseinrichtung zusammenwirken können, wenn das Behälteroberteil den zugeordneten Behälterunterteil in der Schließstellung verschließt.

Günstig ist es, wenn die Sterilisationsstatusdetektionseinrichtung im Behälterinnenraum oder außen am Sterilbehälter angeordnet oder ausgebildet ist. Im oder am Behälterinnenraum kann die Sterilisationsstatusdetektionseinrichtung insbesondere geschützt angeordnet oder ausgebildet werden. Eine Anordnung derselben außen am Sterilbehälter ermöglicht insbesondere das Zusammenwirken derselben auf einfache Weise mit einer Anzeigeeinrichtung oder einer Verschlusseinrichtung des Sterilbehälters.

Vorzugsweise ist die Sterilisationsstatusdetektionseinrichtung ausgebildet zum Detektieren eines Druckgradienten im Behälterinnenraum oder in der Umgebung des Steril behälters. Einen Druckgradienten zu detektieren bietet den Vorteil, ein Ansprechen beziehungsweise eine Funktion der Sterilisationsstatusdetektionseinrichtung optimal auf einen herkömmlichen Sterilisationsvorgang in einem Autoklaven abzustimmen. Bei einem Sterilisationsprozess werden in der Regel mehrere Druckänderungen vollzogen, die insbesondere gegenüber natürlichen Druckschwankungen, beispielsweise wetterabhängigen Druckschwankungen, deutlich größere Druckgradienten erzeugen, so dass sich ein korrektes Durchlaufen eines Sterilisationsprozesses mit einer Sterilisationsstatusdetektionseinrichtung zum Detektieren eines Druckgradienten bestmöglich feststellen lassen kann.

Um eine Druckdifferenz in eine Bewegung des Kolbens umsetzen zu können, ist es günstig, wenn die Kolbenzylinderanordnung einen doppeltwirkenden Zylinder umfasst. Bei einem doppeltwirkendem Zylinder sind normalerweise durch den Kolben zwei Bereiche des Zylinders räumlich voneinander getrennt, die mit jeweils einem Anschluss versehen sind, so dass bei Anlegen unterschiedlicher Drücke an den beiden Anschlüssen der Kolben in die eine oder die andere Richtung in gewünschte Weise bewegbar ist.

Gemäß einer weiteren bevorzugten Ausführungsform der Erfindung kann es vorteilhaft sein, wenn der Zylinder einen Kolben, welcher eine Kolbenstange trägt, und mindestens ein Rückstellglied zum Bewegen des Kolbens in eine Grundstellung umfasst. Auf diese Weise kann sichergestellt werden, dass die Kolbenzylinderanordnung vor Beginn des Sterilisationsprozesses eine definierte Stellung einnimmt, nämlich die, in der der Kolben die Grundstellung einnimmt.

Vorteilhaft ist es, wenn die Kolbenstange entgegen der Wirkung des mindestens einen Rückstellglieds weiter aus dem Zylinder heraus oder in diesen hinein bewegbar ist. Mit dem Rückstellglied, welches beispielsweise in Form eines Federelements, zum Beispiel einer Schraubenfeder, ausgebildet sein kann, lässt sich insbesondere individuell einstellen, welche im Autoklav herrschenden Druckverhältnisse überhaupt eine Bewegung des Kolbens bewirken können. Auf diese Weise lassen sich insbesondere rein mechanisch Grenzwerte von Druckgradienten einstellen, die von der Sterilisationsstatusdetektionseinrichtung zuverlässig bestimmt werden sollen.

Günstig ist es, wenn das mindestens eine Rückstellglied ein Druckglied ist und sich auf einer ersten Kolbenseite des Kolbens abstützt, die die Kolbenstange trägt. So kann beispielsweise der Kolben weiter aus dem Zylinder herausbewegt werden entgegen der Wirkung des Rückstellglieds. Dieses kann insbesondere ein Federelement in Form einer Blatt- oder Schraubenfeder sein.

Alternativ ist es auch denkbar, dass das mindestens eine Rückstellglied ein Zugglied .ist und sich auf einer zweiten Kolbenseite des Kolbens abstützt, die von der Kolbenstange weg weist.

Vorteilhaft ist es, wenn der Zylinder durch den Kolben in einen ersten und einen zweiten Zylinderraum geteilt ist und wenn der Zylinder zwei Anschlüsse umfasst, die jeweils mit einem der beiden Zylinderräume in Fluidverbindung stehen. Wie bereits erwähnt können in Abhängigkeit des Sterilisationsprozesses unterschiedliche Drücke an den Anschlüssen anliegen und auf den Kolben wirken, so dass dieser in die eine oder andere Richtung infolge einer Druckänderung bewegt werden kann.

Um auf einfache Weise Druckgradienten detektieren zu können, ist es günstig, wenn die beiden Anschlüsse unterschiedliche Öffnungsquerschnitte aufweisen. Unterschiedliche Öffnungsquerschnitte ermöglichen es, einem Fluid, insbesondere einem Gas, unterschiedlich schnell in den jeweiligen Zylinderraum hinein und aus diesem heraus zu strömen. Mit anderen Worten kann sich derjenige Anschluss, dessen Öffnungsquerschnitt größer ist, schneller mit dem Fluid füllen beziehungsweise sich schneller entleeren, wenn beide Anschlüsse mit derselben Umgebung, nämlich dem Innenraum des Autoklaven verbunden sind. Eine Druckänderung im Autoklav, also ein entsprechender Druckgradient, führt dann zwangsläufig zu einer Bewegung des Kolbens in die eine oder andere Richtung. Die unterschiedlichen Ein- beziehungsweise Ausströmzeiten führen zumindest temporär zu einer Druckdifferenz zwischen den beiden Zylinderräumen, die insbesondere zur Bewegung des Kolbens genutzt werden kann.

Günstigerweise weisen die Öffnungsquerschnitte der beiden Anschlüsse ein Querschnittsverhältnis von mindestens 2:1 auf, vorzugsweise von mindestens 4:1. Um eine definierte Bewegung des Kolbens in Abhängigkeit eines Druckgradienten im Autoklaven zu erreichen, können die Öffnungsquerschnitte und das Rückstellglied entsprechend aneinander angepasst und aufeinander abgestimmt werden.

Gemäß einer weiteren bevorzugten Ausführungsform der Erfindung kann der Sterilbehälter eine mit der Sterilisationsstatusdetektionseinrichtung gekoppelte Anzeigeeinrichtung zum automatischen Anzeigen des Sterilisationsstatus des Sterilbehälters umfassen. Insbesondere kann die Anzeigeeinrichtung derart ausgebildet sein, dass sie ihre Farbe ändert oder den Sterilisationsstatus in Form von Buchstaben, Zahlen oder Wörtern anzeigt. Denkbar wäre es auch, eine elektronische Anzeigeeinrichtung ohne mechanische Teile zu nutzen, beispielsweise eine LCD- oder LED-Display.

Auf besonders einfache Weise lässt die Anzeigeeinrichtung ausbilden, wenn sie mindestens ein bewegbares oder betätigbares Anzeigeglied umfasst, welches mit der Kolbenstange gekoppelt ist. Infolge einer Bewegung des Kolbens und der an diesem angeordneten Kolbenstange kann so das Anzeigeglied bewegt werden, um infolge einer Positionsänderung desselben einem Nutzer des Sterilbehälters anzuzeigen, ob der Sterilisationsstatus des Sterilbehälters "steril" oder "unsteril" ist. Eine rein mechanisch ausgebildete Anzeigeeinrichtung hat gegenüber einer elektronischen den Vorteil, dass keine elektrische Energie zum Anzeigen des Sterilisationsstatus benötigt wird und zudem Korrosionsprobleme bei der Heißdampfsterilisation vermieden werden können. Wenn das Anzeigeglied und die Kolbenstange unbeweglich miteinander gekoppelt oder einstückig ausgebildet sind, genügt es prinzipiell, nur ein Rückstellglied vorzusehen. Sind das Anzeigeglied und die Kolbenstange beweglich gekoppelt, kann insbesondere vorgesehen sein, dass das Anzeigeglied mit der Kolbenstange lediglich geschoben, nicht jedoch gezogen werden kann. In diesem Fall ist es günstig, zwei Rückstellglieder vorzusehen, eines für die Kolbenstange und eines für das Anzeigeglied.

Günstig ist es, wenn der Sterilbehälter eine mit der Sterilisationsstatusdetektionseinrichtung gekoppelte Verschlusseinrichtung zum Verschließen des Sterilbehälters umfasst. Die Verschlusseinrichtung kann insbesondere derart ausgebildet und/oder angeordnet sein, dass beim Öffnen des Sterilbehälters beispielsweise eine Anzeigeeinrichtung ohne Durchlaufen eines weiteren Sterilisationsprozesses irreversibel von der Statusanzeige "steril" in die Statusanzeige "unsteril" übergeht. Damit kann verhindert werden, was bei rein temperaturaktivierten Sterilisationsstatusdetektionseinrichtungen passieren kann, bei denen nämlich infolge einer Erwärmung auch ohne Heißdampfbeaufschlagung eine Anzeigeeinrichtung auch nach dem Öffnen des Sterilbehälters wieder den Sterilisationszustand "steril" anzeigen kann. Temperaturänderungen haben auf die druckaktivierte Sterilisationsstatusdetektionseinrichtung praktisch keinen Einfluss dahingehend, dass sich ein für die Detektion eines vollständig durchlaufenen Sterilisationsprozesses erforderlicher Mindestwert eines Druckgradienten einstellen kann.

Auf einfache und sichere Weise lässt sich der Sterilbehälter verschließen und in der Schließstellung halten, wenn die Verschlusseinrichtung eine Verschlussklappe umfasst, die am Behälteroberteil oder am Behälterunterteil bewegbar gehalten ist und mit einem Verschlussglied am jeweils anderen Behälterteil in einer Verschlussstellung in Eingriff bringbar ist, wenn der Sterilbehälter die Schließstellung einnimmt. Die Verschlussklappe kann insbesondere verschwenkbar und/oder verschiebbar an einem Teil des Sterilbehälters gehalten oder gelagert sein. Insbesondere kann die Verschlussklappe mit der Anzeigeeinrichtung derart gekoppelt sein, dass beim Überführen der Verschlussklappe von der Verschlussstellung in eine Stellung, in welcher das Behälteroberteil vom Behälterunterteil getrennt werden kann, die Anzeigeeinrichtung automatisch den Sterilisationsstatus von "steril" in "unsteril" ändert, um jegliches Risiko zu vermeiden, dass der Inhalt des Sterilbehälters unsteril werden könnte, ohne dass Nutzer desselben davon Kenntnis erlangen.

Günstig ist es, wenn der Sterilbehälter eine mit der Sterilisationsstatusdetektionseinrichtung gekoppelte Verriegelungseinrichtung zum automatischen Verriegeln der Verschlusseinrichtung umfasst und wenn die Verriegelungseinrichtung mindestens ein Verriegelungsglied umfasst, welches von der Sterilisationsstatusdetektionseinrichtung betätigbar ist, sobald der Sterilisationsstatus "steril" erreicht ist, zum Sichern der Verschlusseinrichtung in der Verschlussstellung.

Besonders einfach und kompakt ausbilden lässt sich der Sterilbehälter, wenn die Sterilisationsstatusdetektionseinrichtung an oder in der Verschlussklappe angeordnet oder ausgebildet ist. Insbesondere kann so eine Bewegung der Verschlussklappe zum Öffnen des Sterilbehälters auf einfache Weise genutzt werden, beispielsweise um den von einer Anzeigeeinrichtung angezeigten Sterilisationsstatus automatisch zu ändern, also insbesondere von "steril" in "unsteril".

Vorteilhaft ist es ferner, wenn die Anzeigeeinrichtung eine erste Halteeinrichtung umfasst, die das Anzeigeglied in einer den Sterilisationsstatus steril anzeigenden Position hält, bis die Verschlusseinrichtung des Sterilbehälters zum Öffnen desselben betätigt wird. Mit anderen Worten ist die Halteeinrichtung derart ausgebildet, dass sie beim Übergang des Sterilisationsstatus von "unsteril" nach "steril" automatisch in eine Halteposition gebracht wird und das Anzeigeglied in der den Sterilisationsstatus "steril" anzeigenden Position hält, bis die Halteeinrichtung infolge einer Bewegung oder Betätigung der Verschlusseinrichtung, beispielsweise der Verschlussklappe, wieder das Anzeigeglied freigibt, so dass die Anzeigeeinrichtung wieder den Sterilisationsstatus "unsteril" anzeigt.

Besonders einfach ausbilden lässt sich die erste Halteeinrichtung, wenn sie mindestens ein erstes Halteglied umfasst, welches den Kolben automatisch in der den Sterilisationsstatus "steril" anzeigenden Position hält.

Günstig kann es ferner sein, wenn das mindestens eine erste Halteglied infolge einer Bewegung des Kolbens von einer ersten Haltegliedrückhaltestellung in eine erste Haltegliedfreigabestellung auslenkbar ist und automatisch nach Erreichen der den Sterilisationsstatus "steril" anzeigenden Position des Kolbens wieder in die erste Haltegliedrückhaltestellung bewegt wird. Das erste Halteglied kann in der Haltegliedrückhaltestellung beispielsweise den ausgelenkten Kolben oder das an diesem gehaltene Anzeigeglied in der den Sterilisationsstatus "steril" anzeigenden Stellung halten.

Damit das erste Halteglied auf einfache Weise wieder von der Haltegliedfreigabestellung in die Haltegliedrückhaltestellung gebracht werden kann, ist es vorteilhaft, wenn das mindestens eine erste Halteglied entgegen der Wirkung eines ersten Haltegliedrückstellglieds von der ersten Haltegliedrückhaltestellung in die erste Haltegliedfreigabestellung auslenkbar ist. Das erste Haltegliedrückstellglied kann insbesondere in Form eines Federelements, beispielsweise einer Schraubenfeder, ausgebildet sein.

Günstig ist es, wenn die Verriegelungseinrichtung eine zweite Halteeinrichtung umfasst, die das mindestens eine Verriegelungsglied in einer den Sterilisationsstatus anzeigenden Position hält, bis die Verschlusseinrichtung des Sterilbehälters zum Öffnen desselben betätigt wird. Mit der zweiten Halteeinrichtung kann somit die Verschlusseinrichtung in einer definierten Position gehalten werden.

Die zweite Halteeinrichtung ist besonders einfach ausbildbar, wenn sie mindestens ein zweites Halteglied umfasst, welches den Kolben automatisch in der den Sterilisationsstatus steril anzeigenden Position hält.

Vorzugsweise ist das mindestens eine zweite Halteglied infolge einer Bewegung des Kolbens von einer zweiten Haltegliedrückhaltestellung in eine zweite Haltegliedfreigabestellung auslenkbar und wird automatisch nach Erreichen der den Sterilisationsstatus "steril" anzeigenden Position des Kolbens wieder in die zweite Haltegliedrückhaltestellung bewegt. Dadurch lässt sich der Kolben auf einfache Weise in einer bestimmten Stellung halten. Damit bildet er quasi ein Gedächtnis des Sterilbehälters, denn durch das zweite Halteglied bleibt er in seiner dem Sterilisationsstatus "steril" zugeordneten Position, und zwar unabhängig insbesondere von Druck- oder Temperaturänderungen in der Umgebung des Sterilbehälters.

Um das mindestens eine zweite Halteglied in einer definierten Position zu halten, ist es vorteilhaft, wenn es entgegen der Wirkung eines zweiten Haltegliedrückstellglieds von der zweiten Haltegliedrückhaltestellung in die zweite Haltegliedfreigabestellung auslenkbar ist. Das zweite Haltegliedrückstellglied kann ebenfalls in Form eines Federelements, beispielsweise einer Schraubenfeder, ausgebildet sein.

Die Funktionsweise und der Aufbau des Sterilbehälters lassen sich weiter vereinfachen, wenn das erste und/oder das zweite Halteglied eine Aufgleitfläche umfassen, um eine vom Kolben ausgeübte Kraft in einer Kolbenbewegungsrichtung auf das erste und/oder das zweite Halteglied in eine Bewegung desselben in einer Haltegliedbewegungsrichtung umzulenken. Beispielsweise kann so ein senkrecht oder im Wesentlichen senkrecht zum Kolben bewegbares erstes oder zweites Halteglied infolge einer Bewegung des Kolbens auf einfache Weise ausgelenkt werden.

Die eingangs gestellte Aufgabe wird ferner bei einem Verfahren der eingangs beschriebenen Art erfindungsgemäß dadurch gelöst, dass, eine Sterilisationsstatusdetektionseinrichtung verwendet wird, die eine Kolbenzylinderanordnung umfasst.

Insbesondere kann es günstig sein, wenn es sich um einen negativen Druckgradient handelt, welcher detektiert wird. Durch das vorgeschlagene Verfahren kann, anders als bei einer rein temperaturüberwachten oder temperaturaktivierten Bestimmung des Sterilisationsstatus sichergestellt werden, dass eine Zuordnung des Sterilisationsstatus "steril" nur erfolgt, wenn auch tatsächlich eine Sterilisation stattgefunden hat. Die bei einem Heißdampfsterilisationsprozess auftretenden Druckschwankungen beziehungsweise Druckgradienten können somit als eindeutiges Indiz dafür genutzt werden, dass der Sterilisationsprozess ordnungsgemäß durchlaufen wurde.

Vorzugsweise wird als Grenzwert ein negativer Druckgradient pro Zeiteinheit in einem Bereich von -500 mbar/min bis etwa -10 bar/min vorgegeben. Beispielsweise kann der Bereich so eingestellt sein, dass er sicher den unvermeidlichen Druckabfall im Autoklaven nach Abschluss des Bedampfungsstadiums erfasst, welcher sich infolge der Evakuierung des Autoklaven zum Austrocknen des Sterilbehälters einstellt.

Vorteilhaft ist es, wenn der Druckgradient während des Sterilisationsprozesses mit einer Druckgradientenmesseinrichtung automatisch bestimmt wird. Diese kann mechanisch oder elektronisch oder elektromechanisch ausgebildet sein. Durch die automatische Bestimmung des Druckgradienten können Fehlbedienungen durch Nutzer vermieden werden.

Vorzugsweise wird der Druckgradient während des Sterilisationsprozesses im Behälterinnenraum oder außen am Behälter bestimmt. In jedem Fall ist es vorteilhaft, die Druckverhältnisse im Inneren des Sterilisationsgeräts zu ermitteln.

Um für einen Verwender des Sterilbehälters und dessen Inhalts auf einfache Weise erkennbar zu machen, ob der Inhalt der Sterilbehälters steril oder unsteril ist, ist es günstig, wenn der Sterilisationsstatus des Sterilbehälters mit einer Anzeigeeinrichtung automatisch angezeigt wird.

Um ein versehentliches Öffnen des Sterilbehälters weitgehend zu vermeiden, ist es vorteilhaft, wenn bei Erreichen des Sterilisationsstatus "steril" eine Verschlusseinrichtung des Sterilbehälters automatisch verriegelt wird. Der Sterilbehälter lässt sich dann nur noch durch gezielte Entriegelung öffnen, um die in ihm befindlichen Sterilgegenstände zu entnehmen.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Teilansicht eines Sterilbehälters;
- Figur 2A:: eine schematische Prinzipdarstellung einer Sterilisationsstatusdetektionseinrichtung in der Stellung "unsteril";
- Figur 2B:: eine schematische Darstellung der Anordnung aus Figur 2A im Sterilisationsstatus "steril"; und
- Figur 3:: eine schematische Darstellung des Druckverlaufs in der Kammer eines Autoklaven während der Sterilisation.

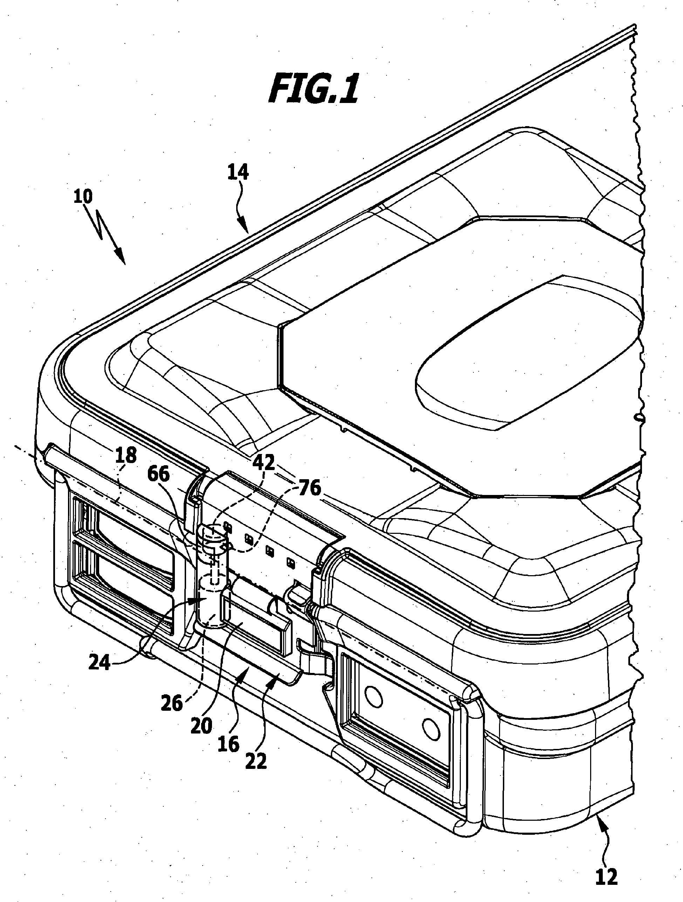

In Figur 1 ist schematisch ein insgesamt mit dem Bezugszeichen 10 bezeichneter Sterilbehälter dargestellt, welcher ein wannenförmiges Behälterunterteil 12 und ein Behälteroberteil 14 zum Verschließen des Behälterunterteils 12 in einer Schließstellung des Sterilbehälters 10 umfasst. Der Sterilbehälter umfasst ferner einen nicht näher bezeichneten Behälterinnenraum zum Aufnehmen sterilisierender Gegenstände. Der Aufbau eines solchen Sterilbehälters 10 ist insbesondere in der DE 201 18 910 U1 beschrieben, deren Offenbarung vollumfänglich in die vorliegende Anmeldung mit einbezogen wird.

Der Sterilbehälter 10 kann am Deckel oder am Boden mit Gasaustauschöffnungen versehen sein, welche mit Keimbarrieren in Form von Filterelementen verschlossen sind. Auch dies ist in der DE 201 18 910 U1 im Detail beschrieben. Alternativ zu Filtern können Gasaustauschöffnungen auch in Form von Ein-und/oder Auslassventilen ausgebildet sein.

Der Sterilbehälter 10 umfasst ferner eine Verschlusseinrichtung 16 zum definierten Verschließen desselben. Die Verschlusseinrichtung 16 umfasst eine um eine Schwenkachse 18 am Behälteroberteil 14 verschwenkbar gehaltene Verschlussklappe 22. Am Behälterunterteil ist ein Verschlussglied 20 angeordnet oder ausgebildet, welches in einer Verschlussstellung mit der Verschlussklappe 22 in Eingriff steht, vorzugsweise klemmend oder rastend. Die Verschlussstellung kann allerdings nur eingenommen werden, wenn der Sterilbehälter 10 die Schließstellung einnimmt, also wie in Figur 1 dargestellt das Behälteroberteil 14 das Behälterunterteil 12 verschließt.

Wie bereits eingangs beschrieben, wird der Sterilbehälter 10 nach Bestücken mit den zu sterilisierenden Gegenständen verschlossen, das heißt er nimmt die in Figur 1 schematisch dargestellte Schließstellung ein und wird dann mit Inhalt in den Autoklaven eingebracht. Die Verschlusseinrichtung 16 nimmt dabei die Verschlussstellung ein.

Um nun einem Verwender des Sterilbehälters 10 zweifelsfrei anzeigen zu können, ob der Sterilbehälter 10 mit seinem Inhalt einen Sterilisationsprozess ordnungsgemäß durchlaufen hat oder nicht, umfasst der Sterilbehälter 10 eine insgesamt mit dem Bezugszeichen 24 bezeichnete, druckaktivierbare Sterilisationsstatusdetektionseinrichtung zum Detektieren eines Sterilisationsstatus des Sterilbehälters 10. Diese ist bei dem in den Figuren dargestellten Ausführungsbeispiel schematisch am Behälteroberteil 14 außen angeordnet, und zwar an der Verschlussklappe 22. Denkbar ist es aber auch, die Sterilisationsstatusdetektionseinrichtung 24 am Behälterunterteil 12 oder im Behälterinnenraum anzuordnen oder auszubilden.

Die Sterilisationsstatusdetektionseinrichtung 24 ist ausgebildet zum Detektieren eines Druckgradienten im Behälterinnerraum oder in einer Umgebung des Sterilbehälters 10. Zur Detektion eines Druckgradienten umfasst die Sterilisationsstatusdetektionseinrichtung 24 eine Kolbenzylinderanordnung 26 mit einem doppeltwirkenden Zylinder 28. Im Zylinder 28 ist in herkömmlicherweise ein Kolben 30 oder eine Kolbenscheibe verschiebbar gelagert. Der Kolben 30 trägt auf einer ersten Kolbenseite 32 eine senkrecht von dieser abstehende Kolbenstange 34, die durch eine Durchbrechung 36 in einer Stirnwand 38 hindurchragt und an ihrem vom Kolben 30 weg weisenden Ende 40 ein zylindrisches Anzeigeglied 42 trägt.

Der Kolben 30 unterteilt den Zylinder 28 in einen ersten Zylinderraum 44 und einen zweiten Zylinderraum 46, die abhängig von einer Stellung des Kolbens 30, unterschiedliche Volumina aufweisen. Im ersten Zylinderraum 44 ist die Kolbenstange 34 umgebend ein Rückstellglied 48 in Form einer Schraubenfeder angeordnet, welche sich einerseits an der Stirnwand 38 und andererseits an der ersten Kolbenseite 32 des Kolbens 30 abstützt. Das Rückstellglied 48 ist in Form eines Druckglieds ausgebildet. Alternativ könnte das Rückstellglied auch in Form eines nicht dargestellten Zugglieds ausgebildet sein, welches einerseits an einer zweiten Kolbenseite 52, die von der Kolbenstange 30 weg weist, und andererseits an der der Stirnwand 38 gegenüberliegenden Stirnwand 54 gehalten ist.

Der Zylinder 28 weist ferner zwei Anschlüsse 56 und 58 auf, die zwischen einer Umgebung des Sterilbehälters 10 und jeweils einem Zylinderraum 44, 46 eine Fluidverbindung herstellen. Ein Durchmesser 60 des Anschlusses 56, welcher mit dem ersten Zylinderraum 44 verbunden ist, ist etwa doppelt so groß wie ein Durchmesser 62 des mit dem zweiten Zylinderraum 46 verbundenen Anschlusses 58. Damit ergibt sich für ein Querschnittsverhältnis der Anschlüsse 56 und 58 ein Wert von etwa 4:1.

Mit der Sterilisationsstatusdetektionseinrichtung 24 ist eine Anzeigeeinrichtung 64 gekoppelt. Diese umfasst das bereits erwähnte Anzeigeglied 42, welches am Ende 40 der Kolbenstange 34 angeordnet ist. Ferner ist an der Verschlussklappe 22 ein Fenster 66 oder andere Art von Öffnung vorgesehen, die von außen erkennen lässt, in welcher Position sich das Anzeigeglied 42 befindet.

Um das Anzeigeglied 42 automatisch in einer Position zu halten, die dem Sterilisationsstatus "unsteril" des Sterilbehälters zugeordnet ist, ist ein weiteres Rückstellglied 68 vorgesehen, das sich einerseits an einer Endfläche 70 des Anzeigeglieds 42 abstützt, welche Endfläche 70 von der Stirnwand 38 weg weist, und andererseits an einer Anschlagfläche 72, die in Richtung auf den Zylinder 28 hin weist. Der Zylinder 28 definiert eine Längsachse 74, wobei die Kolbenstange 34 koaxial zur Längsachse 74 ausgerichtet ist, ebenso wie das Anzeigeglied 42 und das Rückstellglied 68.

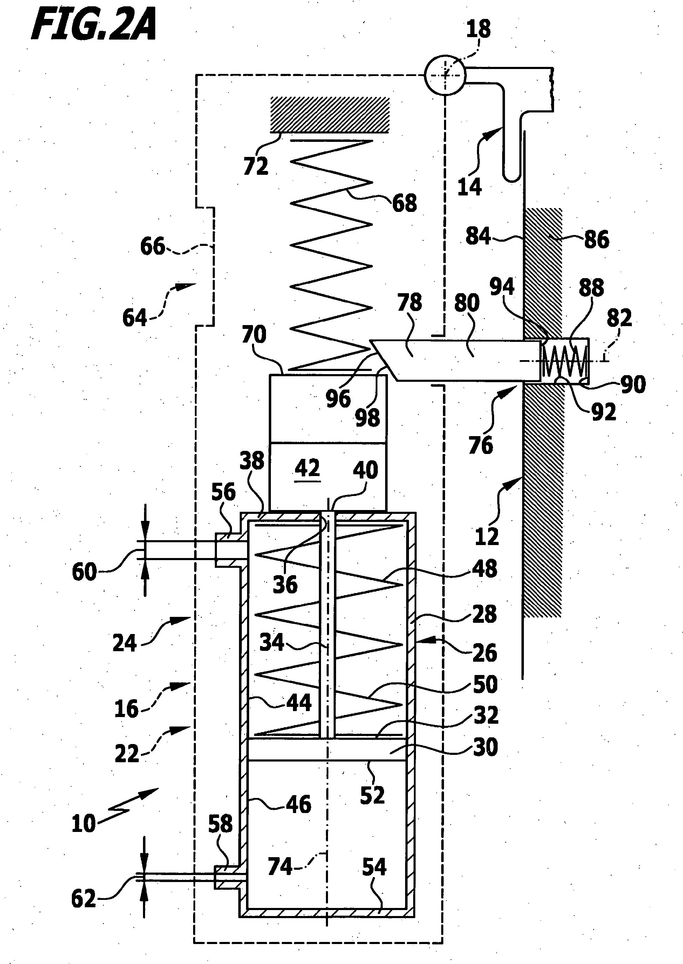

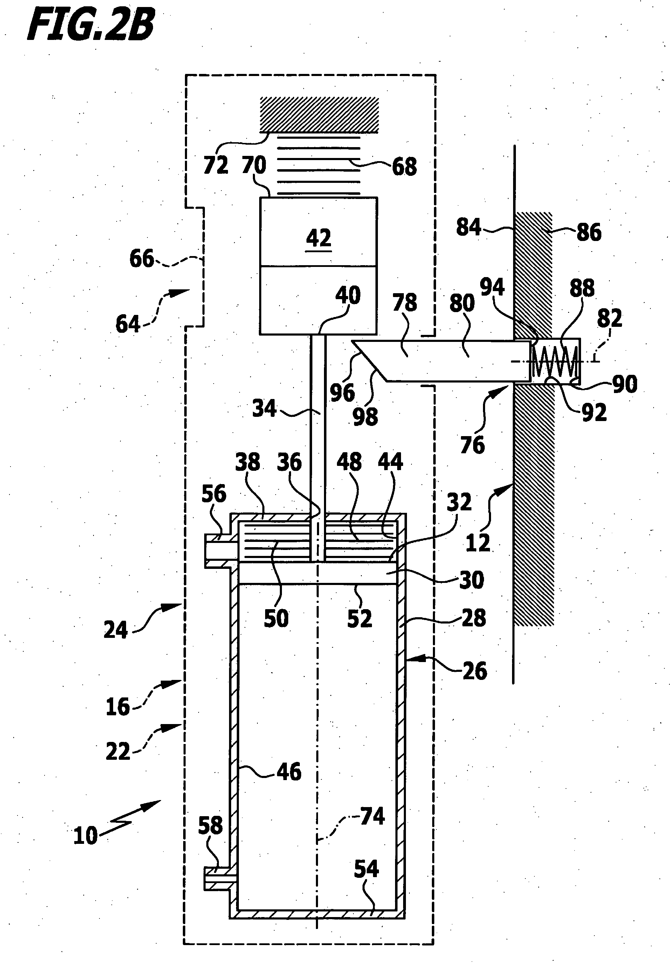

Ferner umfasst der Sterilbehälter 10 eine Halteeinrichtung 76, die das Anzeigeglied 42 in einer den Sterilisationsstatus "steril" anzeigenden Position halten kann bis die Verschlusseinrichtung 16 des Sterilbehälters 10 zum Öffnen desselben betätigt wird. Die Halteeinrichtung 76 umfasst ein Halteglied 78, welches den Kolben 30 beziehungsweise das mit diesem gekoppelte Anzeigeglied 42 automatisch in der den Sterilisationsstatus "steril" anzeigenden Position halten kann. Das Halteglied 78 ist in Form eines zylindrischen Bolzens 80 ausgebildet, welcher eine senkrecht zur Längsachse 74 orientierte Längsachse 82 definiert. Das Halteglied 78 steht teilweise über eine vom Sterilbehälter 10 weg weisende Seitenwand 86 des Behälterunterteils 12 vor und ist parallel zur Längsachse 82 relativ zum Behälterunterteil 12 verschiebbar gehalten. Das Halteglied 78 ist in der Seitenwand 86 des Behälterunterteils 12 mittels eines Haltegliedrückstellglieds 88 gehalten. Dies kann insbesondere in Form einer Druckfeder ausgebildet sein, die einerseits am Boden 90 einer in der Seitenwand 86 des Behälterunterteils 12 ausgebildeten Ausnehmung 92 und andererseits an dem auf dem Boden 90 hin weisenden Ende 94 des Halteglieds 78 gehalten ist. Auf diese Weise kann das Halteglied 78 entgegen der Wirkung des Haltegliedrückstellglieds 88 in Richtung auf den Boden 90 hin bewegt werden. Wird keine Kraft auf das Halteglied 78 ausgeübt, zwingt das Haltegliedrückstellglied 88 das Halteglied 78 in seine in den Figuren 2A und 2B schematisch dargestellte Haltegliedrückhaltestellung, in welcher das Ende 94 vom Boden 90 den größten Abstand aufweist. Ein vom Ende 94 weg weisendes zweites Ende 96 des Halteglieds 78 definiert eine sowohl gegenüber der Längsachse 82 als auch der Längsachse 74 geneigte Aufgleitfläche 98, welche in nachfolgend noch näher beschriebener Weise mit dem Anzeigeglied 42 zusammenwirkt.

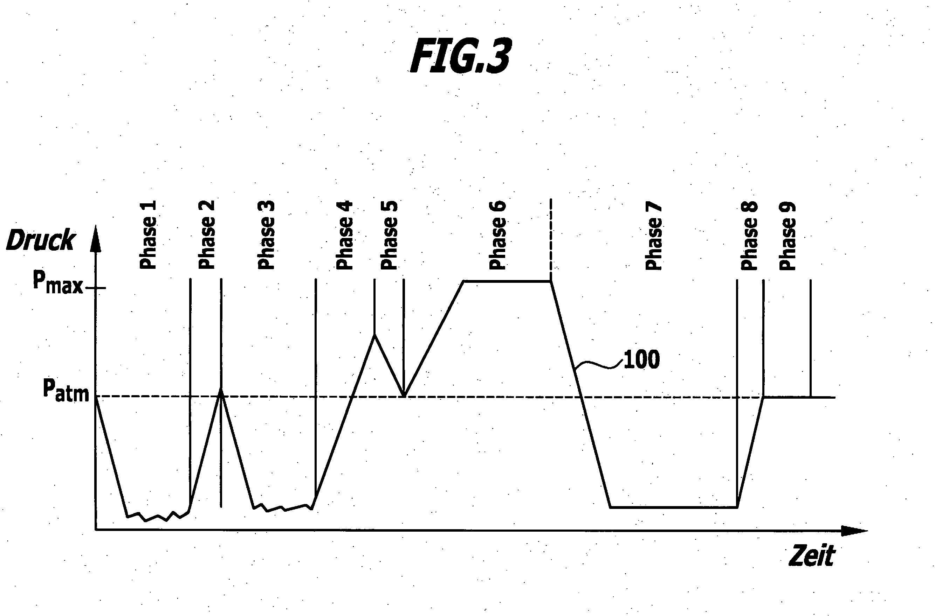

Um die Funktionsweise der druckaktivierbaren Sterilisationsstatusdetektionseinrichtung 24 verstehen zu können, wird zunächst, bezugnehmend auf Figur 3, der zeitliche Druck- und Verfahrensablauf eines typischen Sterilisationsprozesses beschrieben.

Nach Beschicken des Autoklaven mit dem Sterilbehälter 10 wird in Phase 1 der Autoklav evakuiert. Daran schließt sich Phase 2 an, in welcher der Autoklav kurzzeitig durchdampft wird. Dabei steigt der Druck im Autoklav wieder auf Normal- oder Atmosphärendruck pₐₜₘ an. In Phase 3 wird der Autoklav nochmals evakuiert, um in Phase 4 nochmals mit Heißdampf beaufschlagt zu werden. In Phase 4 wird jedoch der Druck im Autoklav etwas über den in der Umgebung des Autoklaven herrschenden Atmosphärendruck erhöht. In Phase 5 wird der nun mit Heißdampf unter Überdruck stehende Autoklav nochmals bis auf Atmosphärendruck evakuiert. Die eigentliche Heißdampfsterilisation erfolgt in Phase 6, in welcher der Sterilisationsraum des Autoklaven mit Heißdampf beaufschlagt wird, und zwar bis zur Prozesstemperatur und zum maximalen Prozessdruck pₘₐₓ. Die maximale Prozesstemperatur und der maximale Prozessdruck werden dann für eine vorgegebene Zeit, beispielsweise 3 bis 60 Minuten, konstant gehalten. In Phase 7 wird die Sterilisationskammer nochmals evakuiert, und zwar bis weit unter Normaldruck. Der Unterdruck wird über eine vorgegebene Zeit gehalten, um den Behälter samt Inhalt zu trocknen. Abschließend wird in Phase 8 der Autoklav wieder belüftet. Der Sterilbehälter kann nach Abkühlen des Autoklaven am Ende von Phase 9 entnommen werden.

Das in Figur 3 schematisch dargestellte Diagramm zeigt den Druckverlauf über der Zeit. Die eigentliche Dampfsterilisation ist jedoch bereits abgeschlossen am Ende der Phase 6. Am Beginn der Phase 7 ergibt sich aufgrund der Evakuierung ein negativer Druckgradient 100. Die Sterilisationsstatusdetektionseinrichtung 24 ist insgesamt so ausgerichtet, dass mit ihr der Druckgradient 100 bestimmt und damit das ordnungsgemäße Ende der Phase 6 und das ordnungsgemäße Durchlaufen des Sterilisationsprozesses festgestellt werden kann.

Befindet sich der Sterilbehälter 10 in der Sterilisationskämmer des Autoklaven, führt der negative Druckgradient 100 dazu, dass der im Zylinder 28 befindliche Dampf aus dem ersten Zylinderraum 44 schneller entweicht als aus dem zweiten Zylinderraum 46, und zwar aufgrund der unterschiedlichen Querschnitte der Anschlüsse 56 und 58. Folge hiervon ist, dass, wie schematisch in Figur 2B dargestellt, der Druck im zweiten Zylinderraum 46 den Druck im ersten Zylinderraum 44 temporär übersteigt, so dass der Kolben 30 entgegen der Wirkung des Rückstellglieds 48 in Richtung auf die Stirnwand 38 bewegt wird. Die Kolbenstange 34 mit dem Anzeigeglied 42 bewegt sich gleichzeitig in Richtung auf die Anschlagfläche 72 hin. Dabei gleitet die Endfläche 70 an der Aufgleitfläche 98 auf und schiebt das Halteglied 78 entgegen der Wirkung des Haltegliedrückstellglieds 88 in Richtung auf die Seitenwand 86 des Behälterunterteils 12 hin. Sobald das Anzeigeglied 42 das Halteglied 78 vollständig passiert hat, wird das Halteglied 78 aufgrund der Wirkung des komprimierten Haltegliedrückstellglieds 88 wieder von der ausgelenkten Stellung, die als Haltegliedfreigabestellung bezeichnet wird, in die Haltegliedrückhaltestellung zurückbewegt.

Nimmt die Druckdifferenz zwischen den beiden Zylinderräumen 44 und 46 wieder ab, werden der Kolben 30 und das Anzeigeglied 42 durch die Wirkung des Rückstellglieds 48 und des Rückstellglieds 68 in Richtung auf die Stirnwand 54 zurückbewegt, also in die Ausgangsstellung. Dies ist jedoch nur so weit möglich, bis das Anzeigeglied 42 an dem die Haltegliedrückhaltestellung einnehmenden Halteglied 78 anschlägt, wie dies schematisch in Figur 2B dargestellt ist. Das Anzeigeglied 42 befindet sich nun auf Höhe des Fensters 66, so dass eine Bedienperson von außen durch das Fenster 66 hindurch das Anzeigeglied 42 erkennen kann. Wird dieses beispielsweise in einer Farbe eingefärbt, zum Beispiel rot oder grün, so wird direkt sichtbar, dass nach Abschluss des Sterilisationsprozesses der Sterilbehälter den Sterilisationsstatus "steril" einnimmt.

Wird die Verschlusseinrichtung 16 nicht betätigt, das heißt die Verschlussklappe 22 nicht aus der Verschlussstellung verschwenkt, so bleibt die Anzeigeeinrichtung 64 unverändert. Sie bildet zusammen mit der Halteeinrichtung 78 quasi ein Gedächtnis des Sterilbehälters 10 und zeigt dessen Sterilisationszustand an.

Wird nun aber die Verschlussklappe 22 geöffnet, das heißt ihr freies Ende von der Seitenwand 86 weg verschwenkt, so wird das Anzeigeglied 42 ebenfalls vom Halteglied 78 weg verschwenkt, so das dieses das Anzeigeglied 42 nicht mehr zurückhalten kann. Folge ist, dass die Rückstellglieder 48 und 68 den Kolben 30 in die in Figur 2A dargestellte Grundstellung zurückbewegen können. Gleichzeitig wird jedoch auch das Anzeigeglied 42 in Richtung auf die Stirnwand 38 hin bewegt, so dass das Anzeigeglied 42 durch das Fenster 66 nicht mehr sichtbar ist. Damit ist für einen Nutzer offensichtlich, dass der Sterilbehälter 10 den Sterilisationsstatus "unsteril" angenommen hat, also eine Sterilität des Behälterinhalts nicht mehr gewährleistet ist.

Vorzugsweise wird jede Verschlussklappe 22 am Behälter mit der beschriebenen Sterilisationsstatusdetektionseinrichtung 24 ausgestattet.

Zeigt die Anzeigeeinrichtung 64 den Sterilisationsstatus "unsteril", ist entweder der Druckgradient 100 während des Sterilisationsprozesses nicht ausreichend gewesen, um die Sterilisationsstatusdetektionseinrichtung 24 zu aktivieren oder an den Verschlussklappen 22 des Sterilbehälters 10 wurde manipuliert.

Ergänzend oder alternativ kann eine der Halteeinrichtung 76 entsprechende weitere Halteeinrichtung vorgesehen sein, um die Verschlusseinrichtung 20 in der Verschlussstellung zu verriegeln. Dadurch lässt sich die Prozesssicherheit weiter erhöhen.

Bei dem oben beschriebenen Verfahren zum Bestimmen des Sterilisationsstatus des Sterilbehälters 10 werden also, während der Sterilbehälter 10 einen Sterilisationsprozess in einem Sterilisationsgerät durchläuft, zumindest relative Druckdifferenzen, denen der Sterilbehälter 10 dabei unterworfen ist, detektiert und dem Sterilbehälter 10 automatisch der Sterilisationsstatus "steril" zugeordnet wird, wenn während des Sterilisationsprozesses ein Grenzwert für einen Druckgradient pro Zeiteinheit überschritten wird, beispielsweise der Druckgradient 100. Vorzugsweise wird als Grenzwert ein negativer Druckgradient pro Zeiteinheit in einem Bereich von etwa -500 mbar/min bis etwa -10 bar/min vorgegeben. Dieser wird mit der Sterilisationsstatusdetektionseinrichtung während des Sterilisationsprozesses mit einer Druckgradientenmesseinrichtung, beispielsweise der Kolbenzylinderanordnung 26, automatisch bestimmt. Der Druckgradient kann während des Sterilisationsprozesses im Behälterinnenraum oder außen am Sterilbehälter 10 bestimmt werden. Der Sterilisationsstatus des Sterilbehälters 10 wird mit einer Anzeigeeinrichtung 64 in der beschriebenen Weise automatisch angezeigt. Optional kann bei Erreichen des Sterilisationsstatus steril die Verschlusseinrichtung 16 des Sterilbehälters 10 automatisch verriegelt werden.

## Patentansprüche

1. Medizinischer Sterilbehälter (10) mit einem Behälterinnenraum zum Aufnehmen zu sterilisierender Gegenstände, welcher Sterilbehälter (10) eine druckaktivierbare Sterilisationsstatusdetektionseinrichtung (24) zum Detektieren eines Sterilisationsstatus des Sterilbehälters (10) umfasst, **dadurch gekennzeichnet, dass** die Sterilisationsstatusdetektionseinrichtung (24) eine Kolbenzylinderanordnung (26) umfasst.

2. Medizinischer Sterilbehälter nach Anspruch 1, **dadurch gekennzeichnet, dass** die Sterilisationsstatusdetektionseinrichtung (24) ausgebildet ist zum Detektieren eines Druckgradienten im Behälterinnenraum oder in der Umgebung des Sterilbehälters (10).

3. Medizinischer Sterilbehälter nach Anspruch 1 oder 2, **dadurch gekennzeichnet, dass** die Kolbenzylinderanordnung (26) einen doppeltwirkenden Zylinder (28) umfasst.

4. Medizinischer Sterilbehälter nach Anspruch 3, **dadurch gekennzeichnet, dass** der Zylinder (28) einen Kolben (30), welcher eine Kolbenstange (34) trägt, und mindestens ein Rückstellglied (48, 68) zum Bewegen des Kolbens (30) in eine Grundstellung umfasst.

5. Medizinischer Sterilbehälter nach Anspruch 4, **dadurch gekennzeichnet, dass** die Kolbenstange (34) entgegen der Wirkung des mindestens einen Rückstellglieds (48, 68) weiter aus dem Zylinder (28) heraus oder in diesen hinein bewegbar ist.

6. Medizinischer Sterilbehälter nach Anspruch 4 oder 5, **dadurch gekennzeichnet, dass** das mindestens eine Rückstellglied (48) ein Druckglied (50) ist und sich auf einer ersten Kolbenseite (32) des Kolbens (30) abstützt, die die Kolbenstange (34) trägt oder dass das mindestens eine Rückstellglied (48, 68) ein Zugglied ist und sich auf einer zweiten Kolbenseite (52) des Kolbens (30) abstützt, die von der Kolbenstange (34) weg weist.

7. Medizinischer Sterilbehälter nach einem der Ansprüche 4 bis 6, **dadurch gekennzeichnet, dass** der Zylinder (28) durch den Kolben (30) in einen ersten und einen zweiten Zylinderraum (44, 46) geteilt ist und dass der Zylinder (28) zwei Anschlüsse (56, 58) umfasst, die jeweils mit einem der beiden Zylinderräume (44, 46) in Fluidverbindung stehen.

8. Medizinischer Sterilbehälter nach Anspruch 7, **dadurch gekennzeichnet, dass** die beiden Anschlüsse (56, 58) unterschiedliche Öffnungsquerschnitte aufweisen.

9. Medizinischer Sterilbehälter nach Anspruch 8, **dadurch gekennzeichnet, dass** die Öffnungsquerschnitte der beiden Anschlüsse (56,58) ein Querschnittsverhältnis von mindestens 2:1 aufweisen, vorzugsweise von mindestens 4:1.

10. Medizinischer Sterilbehälter nach einem der voranstehenden Ansprüche, **gekennzeichnet durch** eine mit der Sterilisationsstatusdetektionseinrichtung (24) gekoppelte Anzeigeeinrichtung (64) zum automatischen Anzeigen des Sterilisationsstatus des Sterilbehälters (10).

11. Medizinischer Sterilbehälter nach einem der voranstehenden Ansprüche, **gekennzeichnet durch** eine mit der Sterilisationsstatusdetektionseinrichtung (24) gekoppelte Verschlusseinrichtung (16) zum Verschließen des Sterilbehälters (10).

12. Medizinischer Sterilbehälter nach Anspruch 11, **dadurch gekennzeichnet, dass** der Sterilbehälter (10) eine mit der Sterilisationsstatusdetektionseinrichtung (24) gekoppelte Verriegelungseinrichtung zum automatischen Verriegeln der Verschlusseinrichtung (16) umfasst und dass die Verriegelungseinrichtung mindestens ein Verriegelungsglied umfasst, welches von der Sterilisationsstatusdetektionseinrichtung (24) betätigbar ist, sobald der Sterilisationsstatus steril erreicht ist, zum Sichern der Verschlusseinrichtung (16) in der Verschlussstellung

13. Medizinischer Sterilbehälter nach Anspruch 12, **dadurch gekennzeichnet, dass** die Verriegelungseinrichtung eine zweite Halteeinrichtung umfasst, die das mindestens eine Verriegelungsglied in einer den Sterilisationsstatus anzeigenden Position hält bis die Verschlusseinrichtung (16) des Sterilbehälters (10) zum Öffnen desselben betätigt wird.

14. Verfahren zum Bestimmen des Sterilisationsstatus eines medizinischen Sterilbehälters (10), welcher einen Behälterinnenraum zum Aufnehmen zu sterilisierender Gegenstände und eine druckaktivierbare Sterilisationsstatusdetektionseinrichtung (24) zum Detektieren eines Sterilisationsstatus des Sterilbehälters (10) umfasst, wobei, während der Sterilbehälter (10) einen Sterilisationsprozess in einem Sterilisationsgerät durchläuft, Druckdifferenzen, denen der Sterilbehälter (10) dabei unterworfen ist, detektiert werden und dem Sterilbehälter (10) der Sterilisationsstatus steril zugeordnet wird, wenn während des Sterilisationsprozesses ein Grenzwert für einen Druckgradient pro Zeiteinheit überschritten wird, **dadurch gekennzeichnet, dass** eine Sterilisationsstatusdetektionseinrichtung (24) verwendet wird, die eine Kolbenzylinderanordnung (26) umfasst.

15. Verfahren nach Anspruch 14, **dadurch gekennzeichnet, dass** bei Erreichen des Sterilisationsstatus steril eine Verschlusseinrichtung (16) des Sterilbehälters (10) automatisch verriegelt wird.

## Claims

1. Medical sterile container (10) having a container interior for accommodating articles to be sterilized, which sterile container (10) comprises a pressure-activatable sterilization status detection device (24) for detecting a sterilization status of the sterile container (10), **characterized in that** the sterilization status detection device (24) comprises a piston-cylinder assembly (26).

2. Medical sterile container in accordance with claim 1, **characterized in that** the sterilization status detection device (24) is configured for detecting a pressure gradient in the container interior or in the environment of the sterile container (10).

3. Medical sterile container in accordance with claim 1 or 2, **characterized in that** the piston-cylinder assembly (26) comprises a double-acting cylinder (28).

4. Medical sterile container in accordance with claim 3, **characterized in that** the cylinder (28) comprises a piston (30) carrying a piston rod (34), and at least one reset member (48, 68) for moving the piston (30) into a basic position.

5. Medical sterile container in accordance with claim 4, **characterized in that** the piston rod (34) is movable further out of the cylinder (28) or into the latter counter to the action of the at least one reset member (48,68).

6. Medical sterile container in accordance with claim 4 or 5, **characterized in that** the at least one reset member (48) is a compression member (50) and is supported on a first piston side (32) of the piston (30), which carries the piston rod (34), or the at least one reset member (48, 68) is a tension member and is supported on a second piston side (52) of the piston (30), which faces away from the piston rod (34).

7. Medical sterile container in accordance with any one of claims 4 to 6, **characterized in that** the cylinder (28) is divided by the piston (30) into a first and a second cylinder space (44, 46), and **in that** the cylinder (28) comprises two connections (56, 58) which are each in fluid connection with one of the two cylinder spaces (44, 46).

8. Medical sterile container in accordance with claim 7, **characterized in that** the two connections (56, 58) have different opening cross sections.

9. Medical sterile container in accordance with claim 8, **characterized in that** the opening cross sections of the two connections (56, 58) have a cross-sectional ratio of at least 2:1, preferably at least 4:1.

10. Medical sterile container in accordance with any one of the preceding claims, **characterized by** an indication device (64) coupled to the sterilization status detection device (24) for automatically indicating the sterilization status of the sterile container (10).

11. Medical sterile container in accordance with any one of the preceding claims, **characterized by** a closure device (16) coupled to the sterilization status detection device (24) for closing the sterile container (10).

12. Medical sterile container in accordance with claim 11, **characterized in that** the sterile container (10) comprises a locking device coupled to the sterilization status detection device (24) for automatically locking the closure device (16), and **in that** the locking device comprises at least one locking member which is actuatable by the sterilization status detection device (24) once the sterilization status sterile is reached, in order to secure the closure device (16) in the closed position.

13. Medical sterile container in accordance with claim 12, **characterized in that** the locking device comprises a second holding device which holds the at least one locking member in a position indicating the sterilization status until the closure device (16) of the sterile container (10) is actuated in order to open the latter.

14. Method for determining the sterilization status of a medical sterile container (10) comprising a container interior for accommodating articles to be sterilized and a pressure-activatable sterilization status detection device (24) for detecting a sterilization status of the sterile container (10), wherein while the sterile container (10) is undergoing a sterilization process in a sterilization apparatus, pressure differences to which the sterile container (10) is thereby subjected are detected, and the sterilization status sterile is allocated to the sterile container (10) if during the sterilization process a limit value for a pressure gradient per time unit is exceeded, **characterized in that** a sterilization status detection device (24) is used, which comprises a piston-cylinder assembly (26).

15. Method in accordance with claim 14, **characterized in that** a closure device (16) of the sterile container (10) is automatically locked when the sterilization status sterile is reached.

## Revendications

1. Conteneur médical stérile (10) présentant un espace intérieur de conteneur destiné à accueillir des objets à stériliser, le conteneur stérile (10) comprenant un dispositif de détection d'état de stérilisation (24) pouvant être activé par la pression pour détecter un état de stérilisation du conteneur stérile (10), **caractérisé en ce que** le dispositif de détection d'état de stérilisation (24) comprend un agencement à piston et cylindre (26).

2. Conteneur médical stérile selon la revendication 1, **caractérisé en ce que** le dispositif de détection d'état de stérilisation (24) est réalisé pour détecter un gradient de pression dans l'espace intérieur de conteneur ou dans l'environnement du conteneur stérile (10).

3. Conteneur médical stérile selon la revendication 1 ou la revendication 2, **caractérisé en ce que** l'agencement à piston et cylindre (26) comprend un cylindre à double effet (28).

4. Conteneur médical stérile selon la revendication 3, **caractérisé en ce que** le cylindre (28) comprend un piston (30), qui porte une tige de piston (34), et au moins un organe de rappel (48, 68) pour déplacer le piston (30) dans une position de base.

5. Conteneur médical stérile selon la revendication 4, **caractérisé en ce que** la tige de piston (34) peut, à l'encontre de l'action dudit au moins un organe de rappel (48, 68), être déplacée plus loin hors du cylindre (28) ou plus loin à l'intérieur de celui-ci.

6. Conteneur médical stérile selon la revendication 4 ou la revendication 5, **caractérisé en ce que** ledit au moins un organe de rappel (48) est un organe de compression (50) et s'appuie sur un premier côté de piston (32) du piston (30), qui porte la tige de piston (34), ou **en ce que** ledit au moins un organe de rappel (48, 68) est un organe de traction et s'appuie sur un deuxième côté de piston (52) du piston (30), qui est opposé à celui où se trouve la tige de piston (34).

7. Conteneur médical stérile selon l'une des revendications 4 à 6, **caractérisé en ce que** le cylindre (28) est subdivisé par le piston (30) en une première et une deuxième chambre de cylindre (44, 46), et **en ce que** le cylindre (28) comporte deux raccords de branchement (56, 58), qui sont en liaison d'écoulement de fluide chacun respectivement avec l'une des deux chambres de cylindre (44, 46).

8. Conteneur médical stérile selon la revendication 7, **caractérisé en ce que** les deux raccords de branchement (56, 58) présentent des sections d'ouverture différentes.

9. Conteneur médical stérile selon la revendication 8, **caractérisé en ce que** les sections d'ouverture des deux raccords de branchement (56, 58) présentent un rapport de sections d'au moins 2:1, de préférence d'au moins 4:1.

10. Conteneur médical stérile selon l'une des revendications précédentes, **caractérisé par** un dispositif de visualisation (64) couplé au dispositif de détection d'état de stérilisation (24), pour la visualisation automatique de l'état de stérilisation du conteneur stérile (10).

11. Conteneur médical stérile selon l'une des revendications précédentes, **caractérisé par** un dispositif de fermeture (16) couplé au dispositif de détection d'état de stérilisation (24), pour assurer la fermeture du conteneur stérile (10).

12. Conteneur médical stérile selon la revendication 11, **caractérisé en ce que** le conteneur stérile (10) comporte un dispositif de verrouillage couplé au dispositif de détection d'état de stérilisation (24), pour le verrouillage automatique du dispositif de fermeture (16), et **en ce que** le dispositif de verrouillage comprend au moins un organe de verrouillage, qui peut être actionné par le dispositif de détection d'état de stérilisation (24), dès que l'état de stérilisation stérile est atteint, en vue de sécuriser le dispositif de fermeture (16) dans la position fermée.

13. Conteneur médical stérile selon la revendication 12, **caractérisé en ce que** le dispositif de verrouillage comprend un deuxième dispositif de maintien, qui maintient ledit au moins un organe de verrouillage dans une position indiquant l'état de stérilisation, jusqu'à ce que le dispositif de fermeture (16) du conteneur stérile (10) soit actionné pour son ouverture.

14. Procédé pour déterminer l'état de stérilisation d'un conteneur médical stérile (10), qui comprend un espace intérieur de conteneur destiné à accueillir des objets à stériliser, et un dispositif de détection d'état de stérilisation (24) pouvant être activé par la pression pour détecter un état de stérilisation du conteneur stérile (10), procédé d'après lequel pendant que le conteneur stérile (10) subit un processus de stérilisation dans un appareil de stérilisation, on détecte des différences de pression auxquelles est soumis le conteneur stérile (10) à cette occasion, et l'on attribue au conteneur stérile (10) l'état de stérilisation stérile, lorsque, pendant le processus de stérilisation, une valeur limite d'un gradient de pression par unité de temps est dépassée, **caractérisé en ce que** l'on utilise un dispositif de détection d'état de stérilisation (24), qui comprend un agencement à piston et cylindre (26).

15. Procédé selon la revendication 14, **caractérisé en ce que** lorsque l'état de stérilisation stérile est atteint, un dispositif de fermeture (16) du conteneur stérile (10) est verrouillé automatiquement.
